# EUROPEAN PATENT SPECIFICATION

(11) **EP 3 775 897 B1**
(45) Date of publication and mention of the grant of the patent: **13.12.2023**
(21) Application number: 19717657.1
(22) Date of filing: 29.03.2019
(51) Int. Cl.: G01N 33/543, G01N 33/68

(54) **BIOMARKER ANALYSIS FOR HIGH-THROUGHPUT DIAGNOSTIC MULTIPLEX DATA**
BIOMARKERANALYSE FÜR DIAGNOSTISCHE MULTIPLEXDATEN MIT HOHEM DURCHSATZ
ANALYSE DE BIOMARQUEURS POUR DONNÉES MULTIPLEX DIAGNOSTIQUES À HAUT DÉBIT

(30) Priority: 29.03.2018 US 201862650162 P
(43) Date of publication of application: 17.02.2021
(73) Proprietor: The United States of America, as represented by the Secretary, Department of Health and Human Services, Bethesda, MD 20892-7660 (US)
(72) Inventor: BERZOFSKY, Jay A., Bethesda, MD 20892 (US); WELSH, Joshua Aden, Bethesda, MD 20892 (US); JONES, Jennifer C., Bethesda, MD 20892 (US)
(74) Representative: Eisenführ Speiser
(86) International application number: PCT/US2019/024975
(87) International publication number: WO 2019/191666

(56) References cited:
- WO-A1-2014/082083
- WO-A1-2014/082083
- WO-A1-2017/077105
- NINA KOLIHA ET AL: "Melanoma Affects the Composition of Blood Cell-Derived Extracellular Vesicles", FRONTIERS IN IMMUNOLOGY, vol. 7, 26 July 2016 (2016-07-26), XP055598962, DOI: 10.3389/fimmu.2016.00282
- CATALINA BURBANO ET AL: "Potential Involvement of Platelet-Derived Microparticles and Microparticles Forming Immune Complexes during Monocyte Activation in Patients with Systemic Lupus Erythematosus", FRONTIERS IN IMMUNOLOGY, vol. 9, 1 March 2018 (2018-03-01), XP055598967, DOI: 10.3389/fimmu.2018.00322
- YE TIAN ET AL: "Protein Profiling and Sizing of Extracellular Vesicles from Colorectal Cancer Patients via Flow Cytometry", ACS NANO, vol. 12, no. 1, 8 January 2018 (2018-01-08), pages 671-680, XP055598973, US ISSN: 1936-0851, DOI: 10.1021/acsnano.7b07782

## Description

### FIELD OF THE DISCLOSURE

The disclosure pertains to the identification of extracellular vesicle (EV) groups and subgroups using multiplex flow cytometry.

### BACKGROUND

Extracellular vesicle (EV) sample characterization can be implemented using flow cytometry. According to one study, some sub-populations of EVs in samples have been identified using a bead-based platform in combination with stimulated emission depletion (STED) microscopy. (Koliha et al., J. Extracellular Vesicles 2015, 5:29975.) While EV-based analysis can provide significant data in bead-based measurements, extraction of useful information from the associated large data sets limits the usefulness of EV-based analyses. Accordingly, improved approaches are needed.

WO 2017/077105 A1 describes a method of characterization of cardiac-derived microvesicles (MV). In a first step of the method, cardiac-derived MV are determined from circulating MV based on the presence of CD172a marker. In a next step, the thus determined (CD172a positive) MV are characterized through determining whether CD235a, CD61, CD144, CD14, CD45, CD73 and/or CD3 markers are present. This characterization leads to sub groups of the cardiac-derived MV such as erythroid derived MV, platelet derived MV, etc. By comparing the amount of MV in the different sub groups to respective control values, efficacy of a therapeutic treatment can be monitored, among others. The determination of sub groups and determination of respective amount can be done by means of multicolor flow cytometry technology (FACS).
WO 2014/082083 A1 describes biomarkers that can be assessed for diagnostic, therapy-related or prognostic methods to identify phenotypes, such as a condition or disease, or the stage or progression of a disease, select candidate treatment regimens for diseases, conditions, disease stages, and stages of a condition, and to determine treatment efficacy. It also describes circulating biomarkers such as microvesicles from a bodily fluid that can be used in profiling of physiological states or determining phenotypes, and corresponding methods of assessing the microvesicles

### SUMMARY OF THE DISCLOSURE

The present invention disclosed herein is defined in appended claim 1, and relates to a method comprising: (i) identifying at least two extracellular vesicle (EV) sample groups based on differing responses in multichannel flow cytometry channel counts for a plurality of samples for each of a plurality of channels, each channel defined by a capture agent and a detection agent, and including at least 10 counts; (ii) selecting a set of channels associated with a selected one of the sample groups based on the identified at least two EV sample groups; and (iii) obtaining multichannel flow cytometry channel counts for a test EV sample for each channel of the set of channels to assess whether the test sample is associated with the selected sample group, wherein the plurality of channels, each channel defined by a capture agent and a detection agent, includes channels identifiable with selected scattered light wavelengths or bands of wavelengths in addition to selected fluorescence wavelengths as well as channels identifiable with scattered light spectra in addition to selected fluorescence spectra, wherein one or more deconvolution algorithms are used to separate optical signals corresponding to different channels. In some examples, the set of channels is obtained based on a labeled representation of a t-distributed stochastic neighbor embedding associated with at least some of the plurality of channels.

Moreover, disclosed but not claimed herein are methods and apparatus that permit determination of EV sample groupings and associated channels defined by combinations of capture agents and detection agents. In some cases, the associated channels are used to determine if a sample should be identified as being in a particular sample grouping. Detection agents, channels, capture agents, as well as sample groupings can be determined to permit selection of groupings for particular targets. In some cases, these groupings can be used to define training sets for use in training neural networks for particular sample assessments. Using neural networks trained in this way, additional or previously acquired data can be further processed to fine tune training sets, or to customize detection agent or capture agent selection. In addition, these selections can identify groupings for which additional characterizations can be done such as RNA-Seq analysis which for large data sets would be prohibitive. Markers, channels, and detection agents can be selected for different applications. For example, for a particular pathology of interest, a suitable bead set can be designed and a simpler analysis implement for this pathology. In some cases, data sets are combined, normalized, and annotated and communication using a wide area network such as the internet so the processor intensive operations can be performed remotely. In the following, methods and apparatus for determining such groupings, using the groupings to establish assays, build training sets for development of neural networks, and/or selecting markers, capture agents, and detection agents are provided. A graphical user interface (GUI) is provided that permits an investigator to rapidly screen large data sets and generate customized data sets based on the screening.

In some examples, methods comprise obtaining multichannel flow cytometry channel counts for a plurality of extracellular vesicle (EV) samples for each of a plurality of channels, each channel defined by a capture agent and a detection agent. With a processor, at least two groups of samples exhibiting differing responses based on the multichannel flow cytometry channel counts are identified. In some examples, a heat map is displayed based on the channel counts for each of the plurality of channels. In further examples, the channel counts for each of the plurality of channels are representable as a stored heat map, and a dendogram is derived from the stored heat map based on a hierarchical clustering associated with the stored heat map. In other examples, the derived dendogram is displayed and the at least two groups of samples are identified based on the derived dendogram. In still further examples, principal component scores are obtained based on the stored heat map and the at least two groups of samples are identified based on the principal component scores. In some examples, the principal component scores are displayed and the at least two groups of samples are identified based on the displayed principal component scores. In still other alternatives, the display of the principal component scores is presented with respect to three principal components. According to other examples, the at least two sample groups are identified based on a t-distributed stochastic neighbor embedding, and in some examples, a channel-labeled representation of the t-distributed stochastic neighbor embedding is displayed.

Systems comprise a flow cytometer configured to produce sample counts for a plurality of samples for each of a plurality of channels defined by a combination of a capture antibody and a fluorophore associated with a detection antibody. A display processor is coupled to receive the sample counts and display an associated heat map and a graphical user interface that provides a set of sample grouping procedures selectable in response to activation of an input device. In some examples, the input device is a keyboard or a pointing device, and the set of sample grouping procedures includes principal component analysis. In some embodiments, the set of sample grouping procedures includes principal component analysis, a t-distributed stochastic neighbor embedding, and an agglomerative hierarchical clustering. In additional examples, the display processor is coupled to the display to display one or more of principal component scores, a dendogram associated with the agglomerative hierarchical clustering, and a representation of the t-distributed stochastic neighbor embedding. According to some examples, the display processor is coupled to the display to display channels associated with at least one sample group established by one of the set of sample grouping procedures.

Diagnostic test methods comprise applying a selected set of reagents and a executing a suitable data analysis method, typically implemented as stored processor-executable instructions, followed by a subsequent assay which identify a specific disease state such as tumor progression; The subsequent assay includes one or more of PCR and RNAseq or other approaches. Test kits that include the selected set of reagents and stored processor-executable instructions can also be provided. In other examples, methods based on sets of reagents and analysis approaches are followed by a subsequent assay which either correlates or is associated with predicted response to a specific treatment. The subsequent assay can include one or more of PCR and RNAseq or other assays,.

The foregoing and other features and advantages of the disclosed technology will become more apparent from the following detailed description of several embodiments which proceeds with reference to the accompanying figures.

### BRIEF DESCRIPTION OF THE FIGURES

The patent or application file contains at least one drawing executed in color. Copies of this patent or patent application publication with color drawing(s) will be provided by the Office upon request and payment of the necessary fee.
FIG. 1 illustrates a representative flow cytometer.
FIG. 2 illustrates a representative set of capture antibodies.
FIG. 3 illustrates a representative method of identifying EV groups and subgroups.
FIG. 4 illustrates a representative heat map associated with bead counts for each of a plurality of channels;
FIG. 5 illustrates a portion of a representative heat map showing channel data for a selected sample population.
FIG. 6 illustrates a representative method of evaluating and grouping EVs.
FIGS. 7A-7B illustrate a representative set of capture antibodies and a plot illustrating groupings of fluorescent response produced by a sample.
FIG. 8 illustrates a representative heat map of flow cytometry data obtained with seven sample populations and 333 channels/population.
FIG. 9 is a dendogram illustrating a representative hierarchical clustering based on the flow cytometry data of FIG. 8.
FIG. 10 is a boxplot based on the flow cytometry data of FIG. 8.
FIG. 11A-11B illustrate a principal component analysis (PCA) based on multiplex flow cytometry data.
FIG. 12 is a representative 3-D depiction of a PCA of a representative flow cytometry data set.
FIG. 13 is a Scree plot associated with PCA.
FIG. 14 illustrates a plot based on a t-distributed Stochastic Neighbor Embedding.
FIG. 15 corresponds to FIG. 14, but provides labels for each marker.
FIG. 16 illustrates a heat map based on correlations.
FIG. 17 illustrates a connectogram.
FIG. 18 illustrates a representative apparatus for identifying EV groups and subgroups.
FIG. 19 illustrates a representative user interface.
FIGS. 20A-20B illustrates separation of EV groups for further analysis of EV RNA analysis.
FIG. 21 illustrates a representative process pipeline for samples associated with oligometastatic, PSMA+ prostate cancer.
FIG. 22 illustrates parallel data acquisition and processing.
FIG. 23 illustrates multiplex bead data import and processing as shown in FIG. 22.
FIG. 24 illustrates bead output analyses as shown in FIG. 22.
FIG. 25 illustrates a graphical user interface for data import, processing and data export.
FIG. 26 illustrates a processing/data display field showing principal component analysis.
FIG. 27 illustrates a processing/data display field showing tSNE analysis.

### DETAILED DESCRIPTION

While the present invention relates to a method as defined in appended claim 1, the disclosure generally pertains to methods and apparatus that permit characterization of EV heterogeneity and quantification of selected EVs, as well as identification of EV groups and subgroups based on, for example, patient responses to particular treatments. In typical examples, multiplex assays are combined with high-resolution single EV flow cytometric methods to establish multiplex-to-single EV analysis methods that permit characterization of a broad range of EV subsets, while also measuring concentration of specific EV populations. In one example, EV repertoire can be correlated with response to cancer treatment. Detection of tumor-associated EVs and detection of EV repertoire changes during treatment can permit personalized, bio-adaptive therapies in a wide range of tumor types. For convenient description, EV groups and subgroups associated with different patient response are differentiated without reference to particular treatment. Division of EVs into subgroups can guide additional EV measurements by, for example, guiding selection of additional capture or detection antibodies, or other sensitizations such as scattering elements or nanotags as discussed below.

As used in this application and in the claims, the singular forms "a," "an," and "the" include the plural forms unless the context clearly dictates otherwise. Additionally, the term "includes" means "comprises." Further, the term "coupled" (including "optically coupled") does not exclude the presence of intermediate elements between the coupled items.

The systems, apparatus, and methods described herein should not be construed as limiting in any way. The disclosed systems, methods, and apparatus are not limited to any specific aspect or feature or combinations thereof, nor do the disclosed systems, methods, and apparatus require that any one or more specific advantages be present or problems be solved. Any theories of operation are to facilitate explanation, but the disclosed systems, methods, and apparatus are not limited to such theories of operation.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed systems, methods, and apparatus can be used in conjunction with other systems, methods, and apparatus. Additionally, the description sometimes uses terms like "produce" and "provide" to describe the disclosed methods. These terms are high-level abstractions of the actual operations that are performed. The actual operations that correspond to these terms will vary depending on the particular implementation and are readily discernible by one of ordinary skill in the art.

In some examples, values, procedures, or apparatus' are referred to as "lowest," "best," "minimum," or the like. It will be appreciated that such descriptions are intended to indicate that a selection among many used functional alternatives can be made, and such selections need not be better, smaller, or otherwise preferable to other selections.

In some examples, acquired data is referred to as corresponding to rows and columns of a matrix, but other representations can be used, and the association of data series with rows or columns can be switched and any particular selection is made for convenient illustration. As used herein, "heat map" refers to a two-dimensional data set of sample data, wherein each of a plurality of samples is associated with values (typically counts) associated with a plurality of channels. Heat map also refers to a visual display of such data. In typical examples, values such as counts are color or grey scale encoded for viewing.

In typical examples, the disclosed methods and apparatus can be used in diagnostic assays (determining the presence or absence of disease), predictive assays (determining a likelihood of responding), or treatment response assays, However, the disclosed technology can be used in other applications as well.

In some examples, color and/or grey scale renderings are used for illustration.

### Flow Cytometry

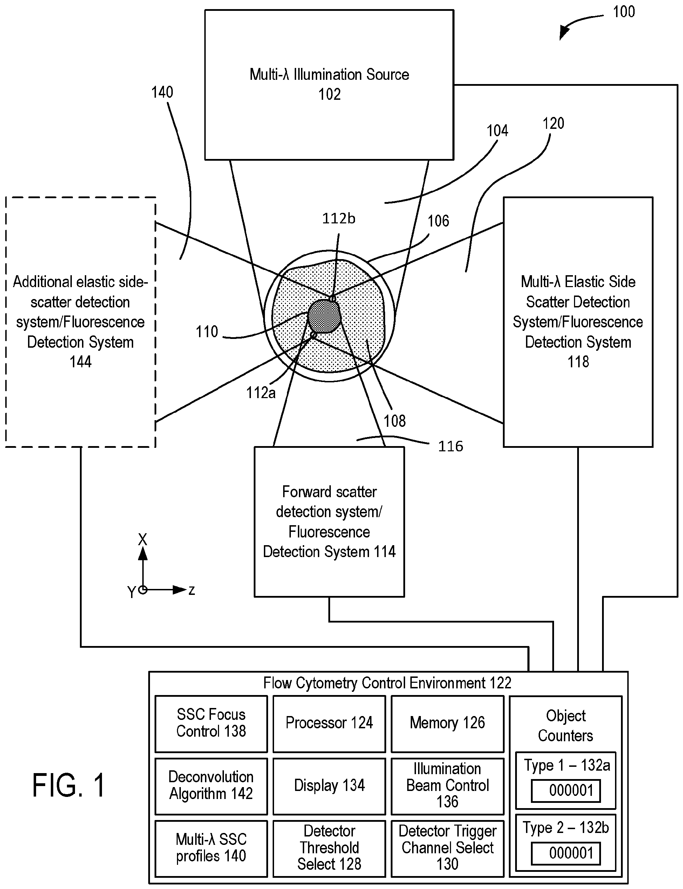

Flow cytometry analysis can be used in multiplex analysis, typically based on measurements of EVs captured by beads to which a set of antibodies is secured. After incubation of beads with EV samples, the captured EVs can be stained using secondary antibodies (referred to herein as detection antibodies) that are associated with respective fluorophores. FIG. 1 shows an example of a microfluidic flow cytometer 100 that includes a multi-wavelength illumination source 102 that produces a multi-wavelength illumination beam 104 and directs the multi-wavelength illumination beam 104 to a microfluidic flow cytometry target 106. In representative examples, the flow cytometry target 106 includes a stream of fluid 108, shown in cross-section such that a stream flows into or out of the plane of FIG. 1, that includes particulates 110 such as extracellular vesicles (EVs) that can become detectable, including singularly detectable, based on capture of the EVs with one or more capture antibodies secured to a bead following by binding to a detection antibody associated with respective fluorophore. In other examples, light is elastically scattered by nanoscale tags ("nanotags") 112a, 112b that are attached to the particulates 110. It will be appreciated that the stream of fluid 108 can be immobile in some examples. The multi-wavelength illumination beam 104 is typically directed to the flow cytometry target 106 perpendicular to the direction of the flow of the stream of fluid 108 and brought to a focus at the flow cytometry target 106. A forward scatter (FSC) detection system 114 is situated opposite the flow cytometry target 106 from the multi-wavelength illumination beam 104 as incident on the flow cytometry target 106 so as to receive a forward scatter detection beam 116 from the flow cytometry target 106 that propagates in the same general direction of the multi-wavelength illumination beam 104.

Molecular nanotags are nano-sized cytometric labels that can be detected individually or quantitatively enumerated based on corresponding intrinsic light scattering properties. Optical apparatus examples herein are capable of collecting spectral scattered light data from multiple wavelength light sources so as to identify different molecular nanotags that can be modular and can be comprised of different nanomaterials, each with identifiable and distinctive light scattering spectral properties across a wide range of wavelengths. In some examples, optical intensity or power values can be detected. Examples measure light scattering at multiple specific wavelengths and enhanced scatter signals are observed that are associated with gold nanomaterials at wavelengths that correspond to the optical properties of gold. In representative examples, plasmon resonance can relate to absorption, and scattering can correspond to a separate phenomenon, and the sum of absorption and scattering is detected so that complex refractive indices are used, including classical refractive index along with the imaginary part which corresponds to the extinction coefficient and accounts for absorption. Such nanotags can be used alone or in conjunction with fluorescent tags, or fluorophores and detection antibodies without nanotags can be used.

In additional examples, patterns of enhanced light scattering power are demonstrated to differ between materials, according to the optical properties, including the refractive index and extinction coefficient. Such differences can be used with multispectral detection methods at selected wavelengths to discriminate laser light and to further increase sensitivity of detection to the point of detecting single molecules, such as molecular nanotags, each with distinct labels. A side scatter (SSC) detection system 118 is situated to receive and detect a multi-wavelength detection beam 120 that propagates generally to the side of the flow cytometry target 106 and the multi-wavelength illumination beam 104, e.g., perpendicular to the direction of the stream of fluid 108 and the multi-wavelength illumination beam 104. In representative examples, the term side-scatter refers to light scattered by a particle suspended in a stream, such as the stream of fluid 108, that is collected from angles typically ranging from 5 to 180 degrees relative to a direction of propagation of light received by the particle from an illumination source. The multi-wavelength detection beam 120 is produced by elastic collisions between the multi-wavelength illumination beam 104 and the particulates 110 and nanotags 112a, 112b of the flow cytometry target 106. In representative examples, the Mie scattering characteristics of the nanotags for different wavelengths or bands of wavelengths can be numerically modeled so that a correspondence between detected scatter and the presence of one or more nanotags in the flow cytometry target 106 can be determined. For example, detected elastic scatter at or near 405 nm can correspond to silver nanotags bound to EVs, and detected elastic scatter at or near 532 nm can correspond to gold nanotags bound to EVs. Thus, the flow cytometry target 106 can be interrogated with the multi-wavelength illumination beam 104 so that different types of nanotags that produce different respective scatter characteristics at different wavelengths, e.g., the nanotags 112a, 112b, can be detected with the side scatter detection system 118. In some examples, multi-spectral side scatter detection with the SSC detection system 118 can be combined with inelastic scatter (Raman) detection or fluorescence detection.

The SSC detection system 118 (and other detection systems) can include or be coupled to a flow cytometry control environment 122 that can include one or more computing devices including a processor 124 and memory 126 coupled to the processor 124. The control environment 122 can include a detector threshold select 128 situated to adjust a signal threshold for detection of scattered light for a selected detector channel of the SSC detection system 118, and a detector trigger channel select 130 situated to select one or more detector channels of the SSC detection system 118 that triggers a detection event based on the signal threshold or thresholds selected with the detector threshold select 128. FSC and SSC data of each detection event can be compared with predetermined SSC/FSC scatter profiles associated with selected objects, such as particulates 110 and/or nanotags 112a, 112b, and one or more object counters 132a, 132b can be incremented based on positive determinations. Fluorescence can also detected.

In some examples, a detector channel that has a least added noise with the addition of the stream of fluid 108 (but without any particulates 110) is selected as a trigger, and a detector threshold for the selected channel is selected to be at or near the noise level associated with the stream of fluid 108. After subsequent interrogation of the stream of fluid 108 containing the particulates 110 and nanotags 112a, 112b with the multi-wavelength illumination beam 104, events associated with the multi-wavelength detection beam 120 can include noise samples that can be compared with particulate-free reference noise to determine the presence or absence of objects in the flow cytometry target 106 that would not be detected with noise settings configured to minimize background noise.

In representative embodiments, the flow cytometry control environment 122 includes a SSC focus control 138 that is coupled to the SSC detection system 118 so as to adjust focus positions for different wavelengths of the multi-wavelength detection beam 120 at one or more respective optical detectors or the multi-wavelength illumination beam 104 at the flow cytometry target 106. Some examples further includes multi-wavelength side-scatter profiles 140, such as wavelength dependent side scatter characteristics (e.g., intensity, power), for one or more nanoparticles, and particularly for a plurality of nanoparticles, so that the detected characteristics of the multi-wavelength detection beam 120 can be compared with the multi-wavelength side-scatter profiles 140 so as to determine the presence of the nanoparticles. In additional examples, one or more deconvolution algorithms 142 are used to separate optical signals corresponding to different nanoparticles.

In different embodiments, various types of the multi-wavelength illumination source 102 can be used, including a plurality of monochromatic lasers and broadband or supercontinuum laser sources. In some examples, an illumination beam control 136 can be used to control timing and/or generation of the multi-wavelength illumination beam 104, based on wavelength selection, detector readiness, etc. In some examples, an additional SSC detection system 144 can be coupled to the flow cytometry target 106 opposite the multi-wavelength detection beam 120 and SSC detection system 118 so as to receive and detect a separate multi-wavelength detection beam 140 comprising light scattered by the flow cytometry target 106. In some example apparatus, one or more of the SSC detection systems 118, 144 can be situated to detect light other than side-scattered wavelengths, such as fluorescence, Raman, or other optical wavelengths and/or optical effects of interest.

The flow cytometry control environment 122 can include software or firmware instructions carried out by a digital computer. For example, any of the disclosed flow cytometry detection techniques can be performed in part by a computer or other computing hardware (e.g., one or more of an ASIC, FPGA, PLC, CPLD, GPU, etc.) that is part of a flow cytometer control system. The flow cytometry control environment 122 can be connected to or otherwise in communication with the multi-wavelength illumination source 102, FSC detection system 114, SSC detection system 118, and additional SSC detection system 144, programmed or configured to control the multi-wavelength illumination beam 104, detection of FSC, SSC, and/or fluorescence and to compare or sort detection beam data to determine the presence or absence of flow cytometry particulates, beads, and/or nanotags. The computer can be a computer system comprising one or more of the processors 124 (processing devices) and memory 126, including tangible, non-transitory computer-readable media (e.g., one or more optical media discs, volatile memory devices (such as DRAM or SRAM), or nonvolatile memory or storage devices (such as hard drives, NVRAM, and solid state drives (e.g., Flash drives)). The one or more processors 124 can execute computer-executable instructions stored on one or more of the tangible, non-transitory computer-readable media, and thereby perform any of the disclosed techniques. For instance, software for performing any of the disclosed embodiments can be stored on the one or more volatile, non-transitory computer-readable media as computer-executable instructions, which when executed by the one or more processors, cause the one or more processors to perform any of the disclosed illumination/detection techniques. The results of the computations and detected optical characteristics of the flow cytometry target 106 can be stored (e.g., in a suitable data structure) in the one or more tangible, non-transitory computer-readable storage media and/or can also be output to a user, for example, by displaying, on a display device 134, number of counted objects, FSC/SSC intensity or power data, fluorescence data, convolved or deconvolved SSC data, channel selection, noise/trigger levels, etc., such as a graphical user interface.

### EV Sample Preparation and Processing

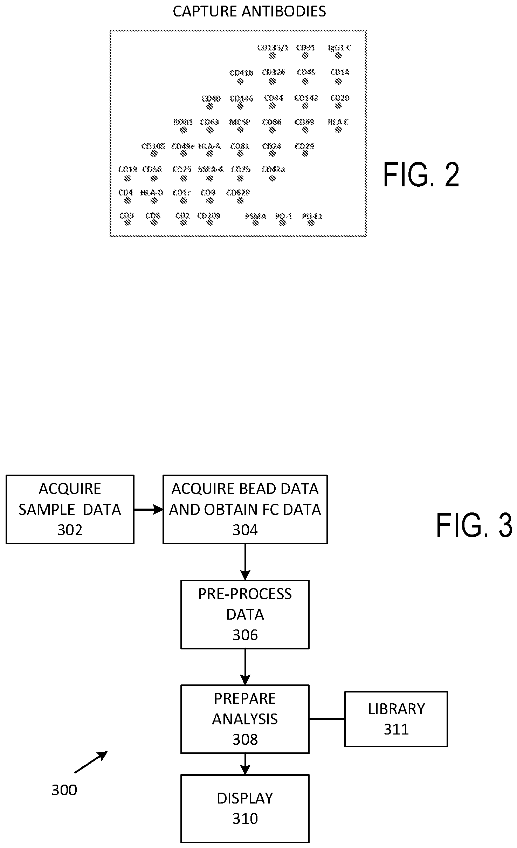

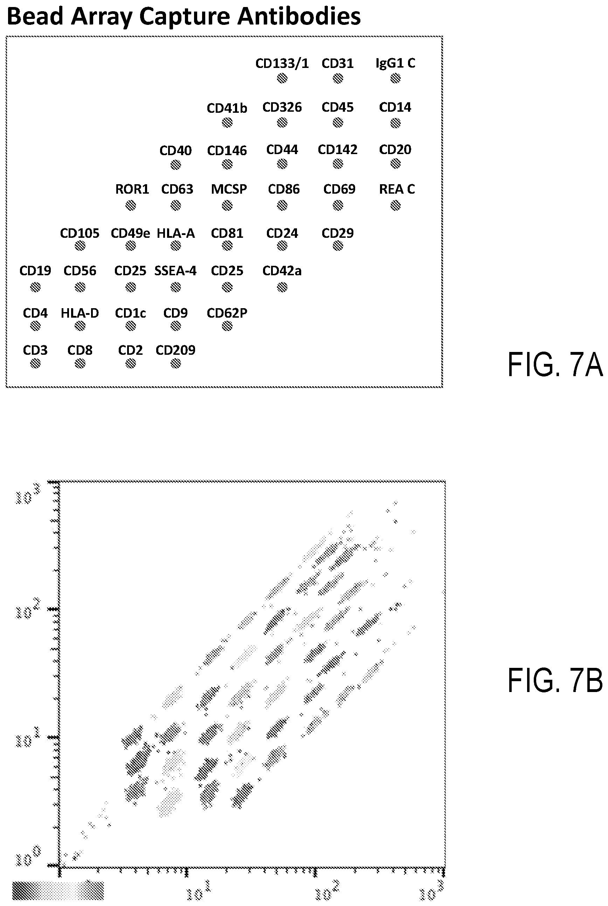

In typical examples, capture antibodies are bound to polystyrene or other beads such as poly(methyl methacrylate) (PMMA) or silica beads. EV specimens are incubated with the beads to promote selective binding of EVs to beads. Unbound EVs are removed via washing. If desired, beads can be dyed prior to incubation to aid in estimating dye concentrations. Various sets of capture antibodies can be used, such as those shown in FIGS. 2 and 7A, and as discussed below, additional capture antibody sets with fewer or more capture antibodies and can be defined based on EV specimen processing. After incubation and washing, beads with bound EVs are exposed to one, two, or more detection antibodies that are associated with respective fluorophores. For convenience, each combination of a fluorophore and a capture antibody is referred to as defining a channel.

### Multiplex Analysis Overview

In typical examples, 40-100 (or more or fewer) capture antibodies are used, and 4-10 detection antibodies with associated fluorophores are used so that a number of channels ranges from 160 to 500; in other example, fewer or more channels are defined. Thus, flow cytometric evaluation of EV populations tends to produce large data set. In a particular example, 39 capture antibodies and 3 detection antibodies are used for each EV sample population, so that acquired data is associated with about 120 different fluorescence response values. If desired, scatter data such as side scatter (SSC) and forward scatter (FSC) can be used with or without nanotags. If a sample population is to be evaluated, each sample will be associated with corresponding response values, and a total data set for the set of samples will included a large number of embedded response values. Methods for extracting practical results and for grouping samples from these complex data sets are required. FIGS. 2 and 7A show representative sets of capture antibodies but other (arbitrary) sets can be selected. In some cases, one or more subsequent sets are chosen based on analyses conducted according to evaluations using an initial set.

### EV Grouping Overview

FIG. 3 is a block diagram of a representative method 300 of processing and grouping EV sample populations. At 302, sample data is acquired, such as whether the sample is associated with response to treatment, non-response to treatment, or lack of treatment. At 304, flow cytometry bead (or other tag) data is acquired, such as capture antibodies and detection antibodies used, along with histograms of numbers of counts per channel defined by beads or nanotags. Representative data acquisition parameters include sensitized bead characteristics, numbers and identifications of capture antibodies, numbers and identifications of detection antibodies, and normalization or control values and processes used to adjust data such as to correct for fluorescence spectra overlap (generally referred to as compensation). At 308, flow cytometry measurement data obtained after pre-processing at 306 to, for example, normalize or reorder, is subjected to one or more analyses to permit identification of EV groups and subgroups. For example, heat maps can be produced, or principal component analysis (PCA) applied to some or all portions of the acquired data. In some cases, data portions associated with low counts are discarded. In some examples, multiple different analyses are performed that permit identification of groups or subgroups. While groupings or subgroupings can be produced at 308, some or all analyses are displayed at 310 either for use in group selection upon visual inspection by a user or to confirm group selection. A graphical user interface can be displayed as well so that a user can confirm, modify, reject, restart, or end analysis. In some examples, preparing analyses at 308 is based on obtaining computer-executable instructions stored in a non-transitory computer readable medium that define a library 311. If desired, groupings can be stored, and FC data discarded.

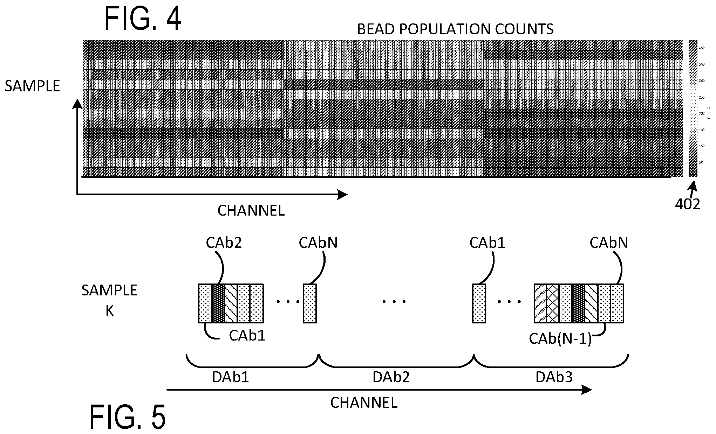

FIGS. 4-5 illustrate representative flow cytometry data. Referring to FIG. 4, a number of beads detected associated with each channel (referred to as a "count") is color-encoded for display. For convenient reproduction, the color mapping can be shown in grey scale, and a color/grey scale assignment of counts is shown at 402. As shown in FIG. 4, a number of counts ranges from zero to about 500. Each row of FIG. 4 thus displays counts for each channel for each sample population. The data presentation of FIG. 4 is referred to as a heat map. FIG. 4 illustrates fourteen samples (shown in fourteen rows), and each row is associated with 200 channels (i.e., 40 capture antibodies and 5 detection antibodies). In most examples, small numbers of counts, such as less than 10, 7, or 5 do not generally provide reliable indicators of sample characteristics, and channels with such low counts are not used in subsequent analysis.

FIG. 5 shows a single row of a representative heat map for a K^{th} sample such as the heat map of FIG. 4. In this example, N capture antibodies CAb1 ... CAbN are used along with three detection antibodies (DAb1, DAb2, DAb3), defining 3N channels. Additional channels associated with scatter or other tags can be used, but are not shown in FIG. 5. Shading of each channel is used to illustrate count/channel.

### Multiplex Bead Processing

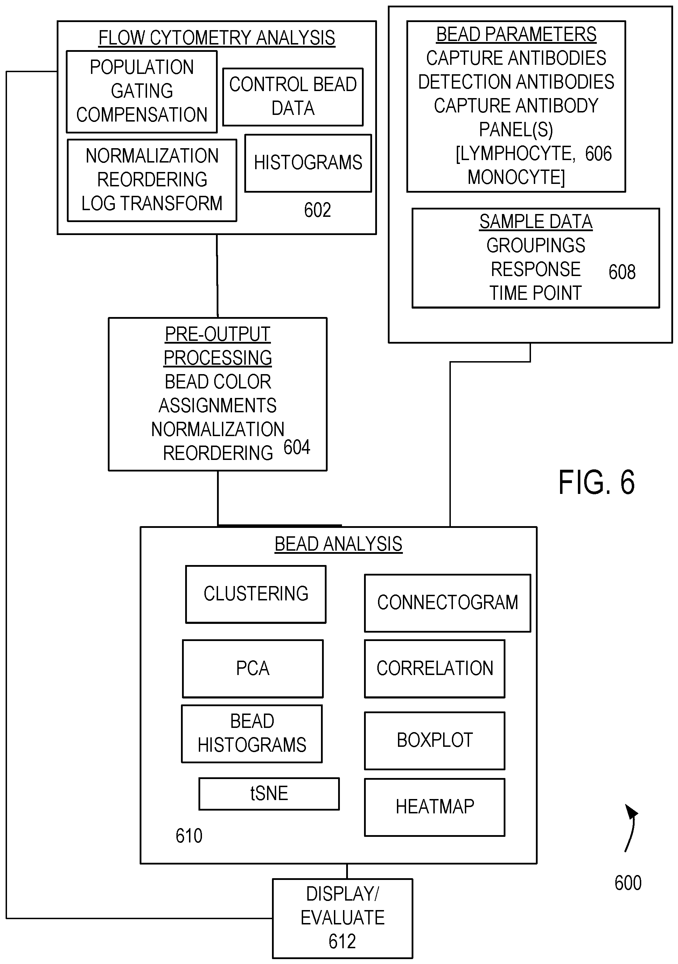

FIG. 6 illustrates a method 600 of obtaining FC data and processing the acquired FC data for identification of groups and subgroups. At 602, FC data is acquired and processed. The acquired data (counts) are assigned to channels based on capture and detection antibodies (and associated fluorophores) that are used. FC data associated with control beads can also be obtained and used to correct for non-specific binding. Data can be normalized, channels reordered, and counts recorded based on logarithm of actual count numbers. Population gating can be applied along with compensation to reduce the effects of fluorophore spectral overlap. In some cases, count histograms are produced. At 604, FC data sets for a plurality of samples are combined to produce a data matrix such as a matrix in which each row is associated with a sample, and each column contains a numerical value associated with a number of counts in a particular channel. The combined data can be suitably normalized or reordered to group similar sample populations, if desired.

Bead and sample characteristics can be stored for used in FC data acquisition, analysis, and reporting of results such as groups or subgroups. For example, beam parameters are stored at 606 and include capture antibodies and detection antibodies and their associated fluorophores. In some cases, identifiers of sets (panels) of capture antibodies are included. Sample data such as sample groupings, responses exhibited by one or more specimens in a sample or sample grouping, and time point associated with a sample treatment are stored at 608.

At 610, one or more procedures can be applied to find EV groupings and subgroupings. Typically, a selection of such procedures is made by a user with a graphical user interface, and results of such analyses are displayed. However, in some examples, results are forwarded to a clinician or other destination via a network, and analysis results are not displayed locally. For example, a heat map can be generated or a hierarchical of other clustering procedure can be applied to identify related samples. In other examples, correlation maps, boxplots, principal component analysis (PCA), t-distributed stochastic neighbor embedding (tSNE) analysis, or heat maps are produced, and associated tabular data, graphics, or other characteristics of a particular analysis that may be helpful to a user are displayed at 612. Examples of these evaluations are discussed below. Based on these evaluation, addition FC data can be acquired at 602 using the same or different antibody panels, or a response evaluated.

FIGS. 7A-7B illustrate a set of capture antibodies and associated FC data, respectively, illustrating response groupings associated with each of the capture antibodies of the set. Individual responses are apparent.

### Sample Analyses

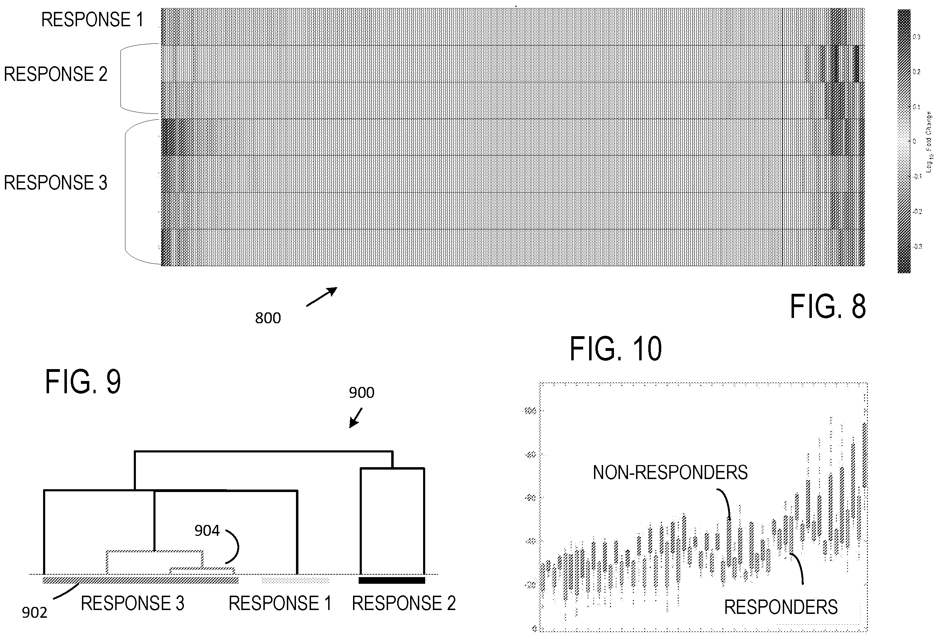

Referring to FIG. 8, a heat map 800 includes seven rows and displays counts for seven samples associated with 333 channels defined by 37 capture antibodies and 9 secondary (detection) antibodies (i.e., 9 phenotypes). In this example, the detection antibodies are a CD9, CD63, a CD81 mixture, HLA-ABC, HLA-E, CD117, CD11b, CD33, CD40, CD3, and CD16. Samples associated with three different treatments are shown, one associated with a first treatment (Response 1), two associated with a second treatment (Response 2), and four associated with a third treatment (Response 3). In the example of FIG. 8, channels displayed in the central portion of the heat map 800 are associated with low to very low counts, and the associated count data may not be used. Inspection of the heat map 800 permits identification of channel response differences among the three treatments, and Response 2 appears to be most different from Response 3, and Response 2 appears more similar to Response 1 than to Response 3.

While presentation of a heat map permits estimation of suitable groupings, groupings can be determined without user inspection (or user inspection can be aided) based on agglomerative hierarchical clustering as illustrated in a dendogram 900 shown in FIG. 9. Selected data associated with each sample is used to represent a location in a multidimensional space, and distances between such points are determined. In a typical example, Euclidean distances are used, but other distance metrics can be used such as Euclidean square or Manhattan distances. Intermediate clusterings can be shown as well. In FIG. 9, Responses 1 and 2 are associated with a single cluster, while Response 3 is associated with a bottom-most cluster 902 and an intermediate cluster 904, indicative of variability within Response 3. The example of FIG. 9 shows results of agglomerative hierarchical clustering on a sample set, but similar clustering can be applied based on detection antibodies as well.

FIG. 10 illustrates a boxplot showing differences in secondary antibody staining intensity for each bead based on groups allocated by a user. In the example of FIG. 10, groupings correspond to non-responders (Responses 1 and 3) and responder (Response 2).

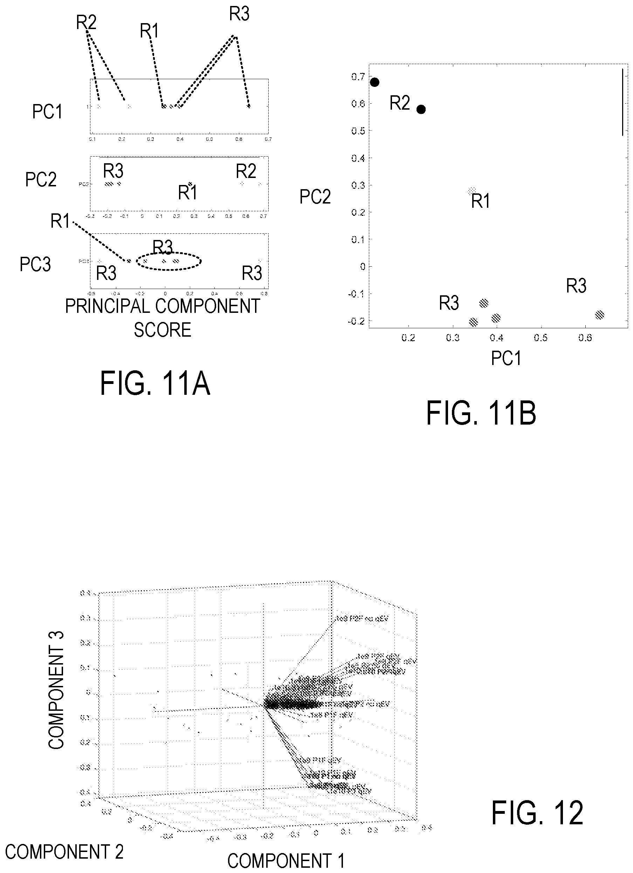

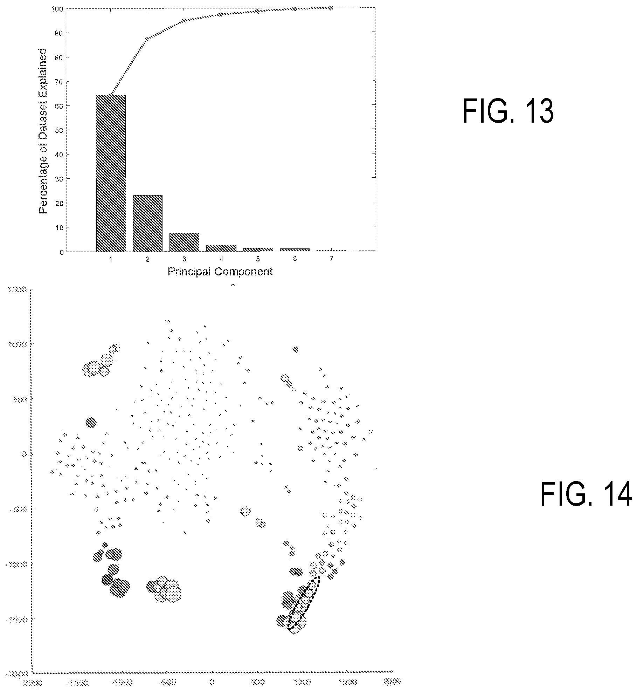

In some examples, PCA is used for determination of groupings. FIG. 11A illustrates unidimensional PCA plots for the first three principal components of a representative PCA of FC data. Sample populations are noted as R1, R2, and R3. FIG. 11B is a 2-dimensional PCA plot showing combinations of the first 3 principal components. FIG. 12 is a 3-dimensional PCA plot which can be displayed as a rotating graph. Variable coefficients are shown as relative points with principal components shown with sample labels. Variable coefficients relative to unique sample clusters in this visualization can be used to identify unique channels of subsets. Extension of the labels from the origin indicate associated principal components, and groupings shown how data variability is provided by the three principal components used in FIG. 12. Principal axis directions can be rotated to obtain additional views. For convenience, such a plot can be referred to as a "labelled PCA" plot in view of the use of channel labels. FIG. 13 is a Scree plot demonstrating the percentage of variability within results accounted for by each principle component.

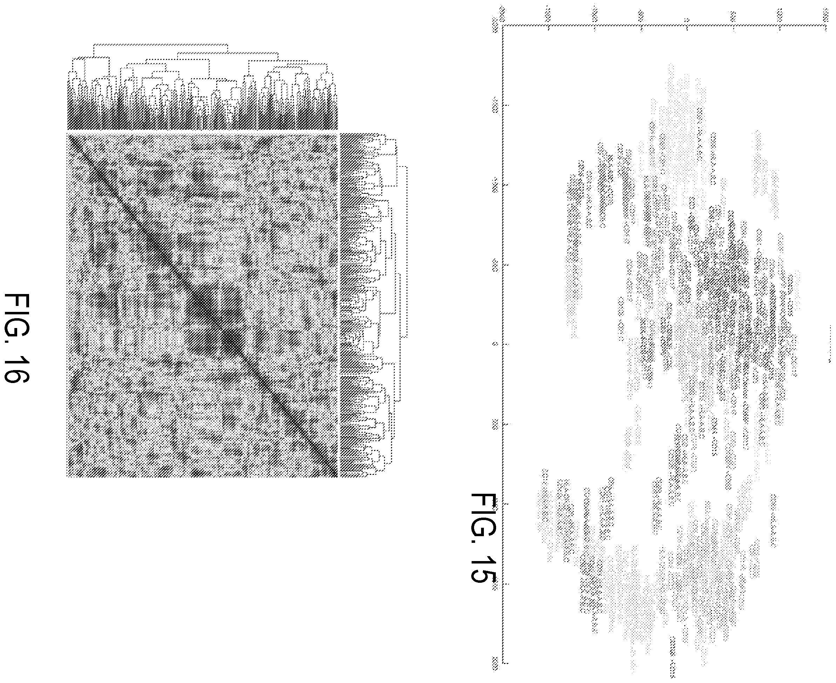

In yet another example illustrated with reference to FIG. 14, t-distributed Stochastic Neighbor Embedding (tSNE) is used to identify groupings. Each data point in FIG. 14 corresponds one combination of one capture antibody (of a set of 37) and one detection antibody (of a set of 9). In FIG. 14, multidimensional multiplexed FC is mapped to two dimensions. In such a mapping, bigger marker sizes indicate more variation of phenotype expression in the dataset. Typically, each detection antibody is assigned a respective color so that determination of variability associated with an antibody is revealed by the graph of FIG. 14. FIG. 15 is a duplicate of the tSNE plot of FIG. 14 with labels for each marker. The labels of FIG. 15 use the same coloring as in FIG. 14. Clustering of markers indicates those that are related - likely increasing and decreasing synchronously, allowing identification of markers for downstream analysis/investigation. The representation of FIG. 15 can be referred to as "labeled" in view of the direct use of channel labels.

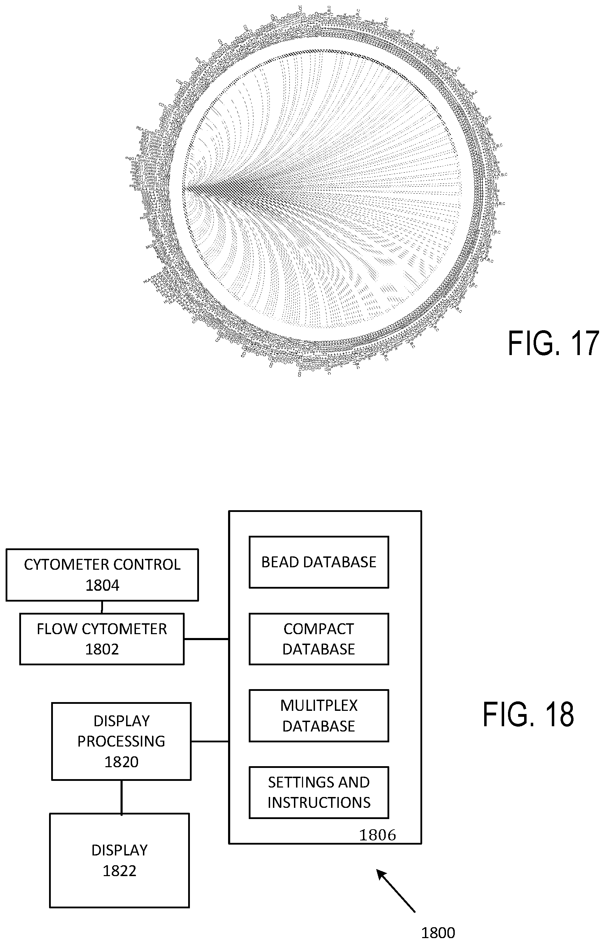

FIG. 16 illustrates a heat map indicating correlation of markers with one another, typically using color. In a grey scale example such a grey scale rendition of FIG. 16, darker regions are associated with larger correlations. A connectogram illustrated schematically in FIG. 17 can be used to link markers (channels) showing significant correlations with one another for identification of targets for further analysis and investigation.

### Representative Multiplex System

Referring to FIG. 18, a system 1800 includes a flow cytometer 1802 that is coupled to a cytometer controller 1804 that regulates fluid flows, data acquisition, analysis, and output of acquired data. The flow cytometer 1802 is typically coupled to a non-transitory computer readable storage medium 1806 that stores bead information (such as capture and detection antibody characteristics), multiplexed data such as histogram data, instrument settings and processor-executable instructions, and processed data (typically compacted as a result of processing) in respective memory portions. In some cases, some or all such data or instructions are obtained or stored via a network connection to a local area or wide area network. In most cases, a display controller 1820 is coupled to a display 1822 so that processed or raw data, instrument settings and instructions, or other information can be provided to a user.

### Representative User Interface

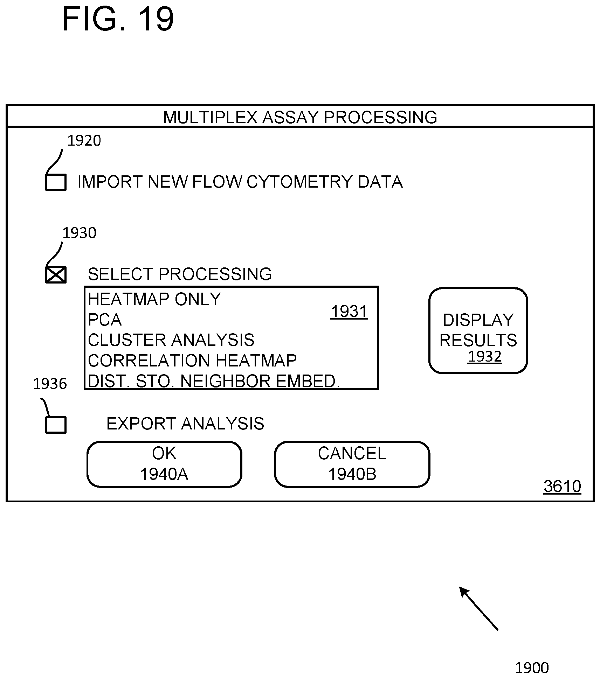

FIG. 19 shows a screen shot 1900 of an exemplary user interface for FC data acquisition, control, and processing to identify groups and subgroups. In the example, import of new data is selected with a checkbox 1920 and selection an analysis method is signaled by selecting a checkbox 1930. A menu box 1931 lists available methods that are selectable by, for example, highlighting with a pointing device such as a mouse. Alternative, a drop down menu can be provided. In some cases, selection of a particular process initiates a user to establish process control. A button 1932 is provided to indicate that analysis results are to be displayed; typically an additional menu is provided for selection of preferred display results. A checkbox 1936 is selectable for output of analytical results such a graphical representations (e.g., heat maps, Scree plots, connectograms) or data associated with such representations. OK and Cancel functionality are provided by the buttons 1940A and 1940B.

### Analysis of EV Cargo in Selected Subsets

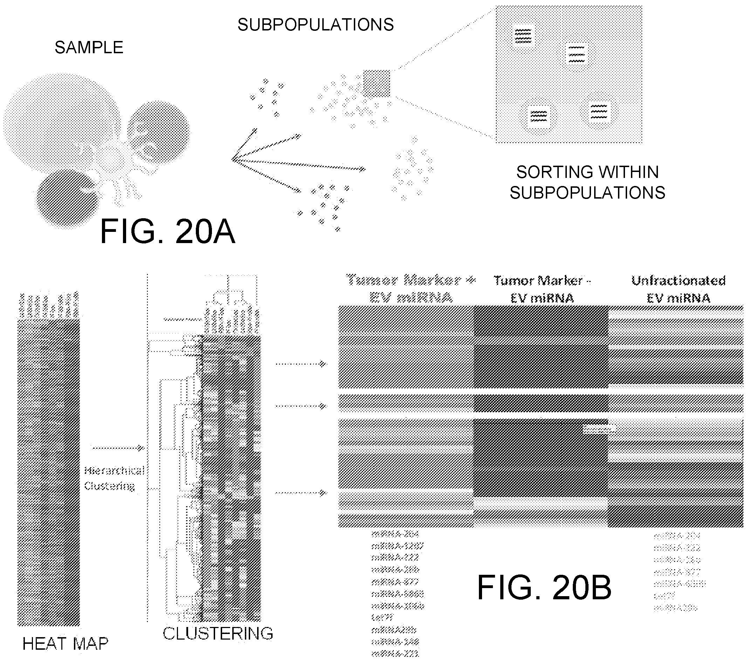

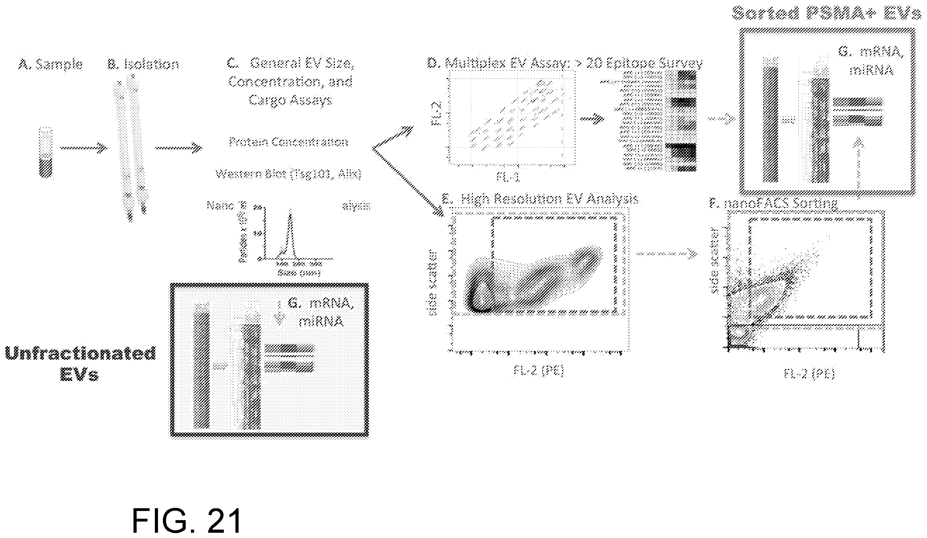

In the examples above, selection of specific sample groups and subgroups allows these groups and subgroups to be sorted and analyzed separately in subsequent assays, such as RNA or DNA sequencing, mutation analysis, or molecular colocalization studies. FIGS. 20A-20B shows a schematic diagram (A) and screen shot of an example of miRNA data (B), wherein groups of EVs isolated based on a tumor-associated marker, Prostate Specific Membrane Antigen, were sorted and analyzed for their miRNA content. The miRNA profiles of the different groups of EVs (FIG. 20B) demonstrate an 11 miRNA signature, including 10 miRNAs previously associated with aggressive prostate cancer cells, whereas the miRNA signature of interest is not clearly represented in the unsorted (total) EV population. FIG. 21 more fully illustrates a process pipeline for use in obtaining such groups and subgroups.

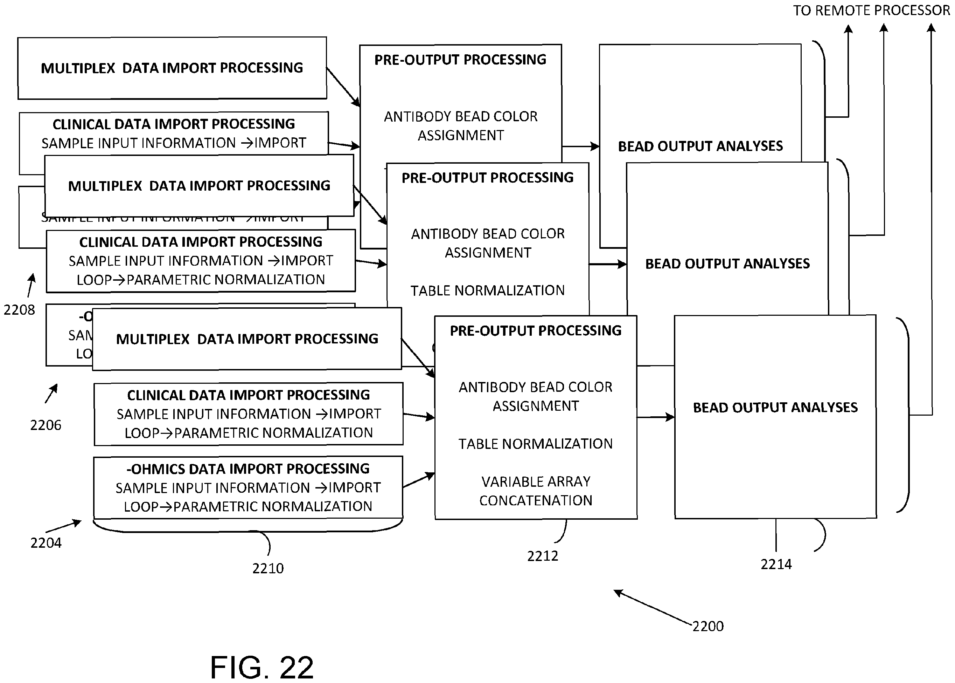

Referring to FIG. 22, parallel data acquisition and processing 2200 is shown by representative data flows 2202, 2204, 2206 that produce outputs that can be directed to the cloud or other network for remote processing. In the data flow 2202, data import is carried out as indicated at 2210 and includes acquiring sample information, importing sample data, and normalization of multiplex data, clinical data, and omics data. At 2212, the imported data from 2210 is processed based on antibody color assignments and data is normalized and concatenated. The processed data is then subject to bead output analysis and then couple to a remote processor. Multiple data flows can executed in parallel, and three are shown for convenient illustration.

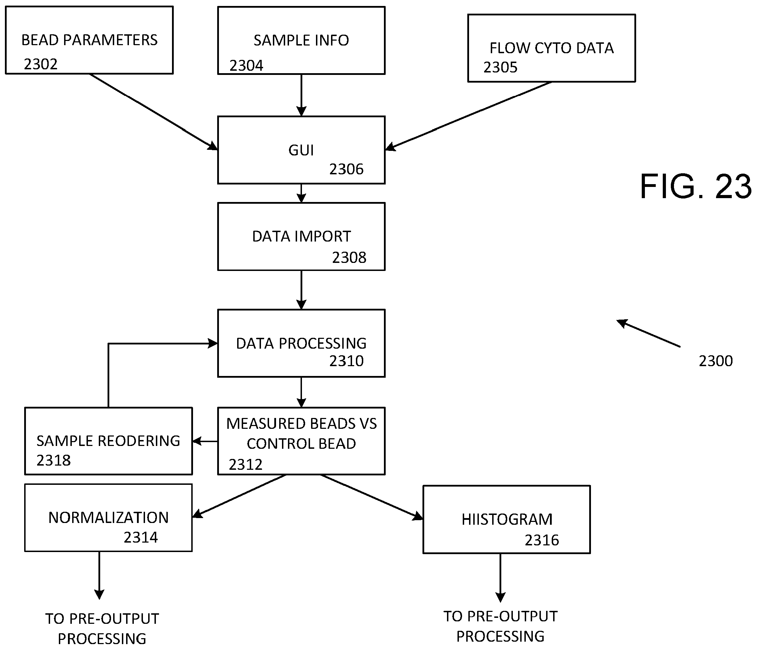

Multiplex bead data import and processing 2300 is illustrated in FIG. 23. A GUI 2306 can be used to control reception of bead parameters 2302, sample information 2304, and flow cytometry data 2306. At 2308, data is imported as selected with the GUI 2306 and then processed at 2310. At 2312, measured and control bead data is compared, and then normalized at 2314 and/or used to generate histograms at 2316. Normalized data and/or histograms are then coupled for pre-output processing as shown in FIG. 22. In some cases, samples are reordered at 2318 for additional data processing at 2310 and bead comparison at 2312.

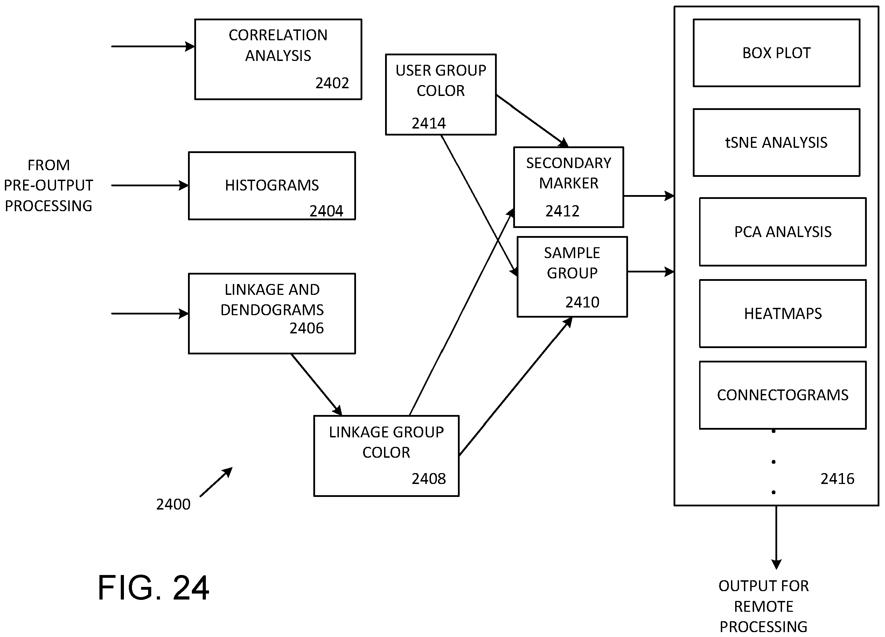

FIG. 24 illustrates a representative arrangement for bead analysis such as shown in FIG. 22. Data from pre-out processing (such as at 2212 in FIG. 22) is coupled to provide correlation analysis at 2402, histograms at 2404, and dendograms at 2406. Linkage group colors are provided at 2408 and user color groups can be defined at 2414. Colors can be associated with secondary markers at 2412 and samples or sample groups at 2410. At 2416, data is selectively processed by generating a boxplot, performing tSNE or PCA analysis, and/or generating heatmaps and/or connectograms. Other types of analysis can be used as well. After analysis, data can be exported for further data analysis or to request acquisition of additional data such as omics data.

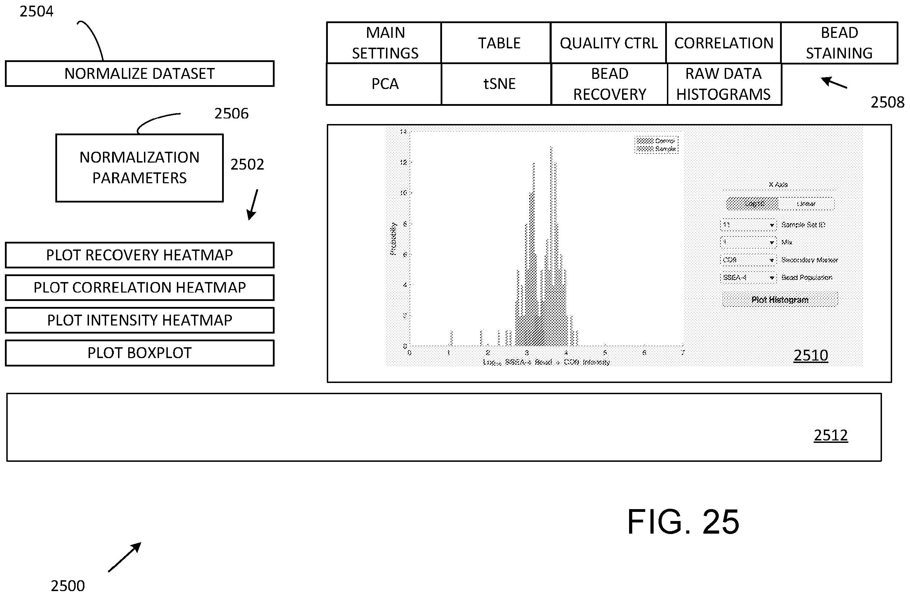

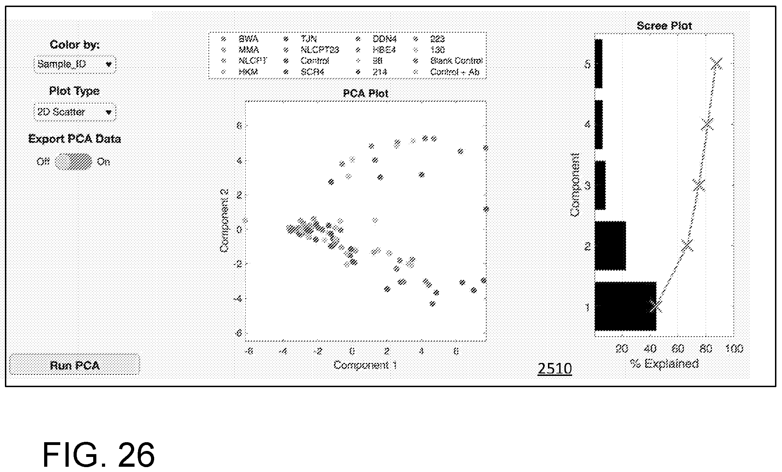

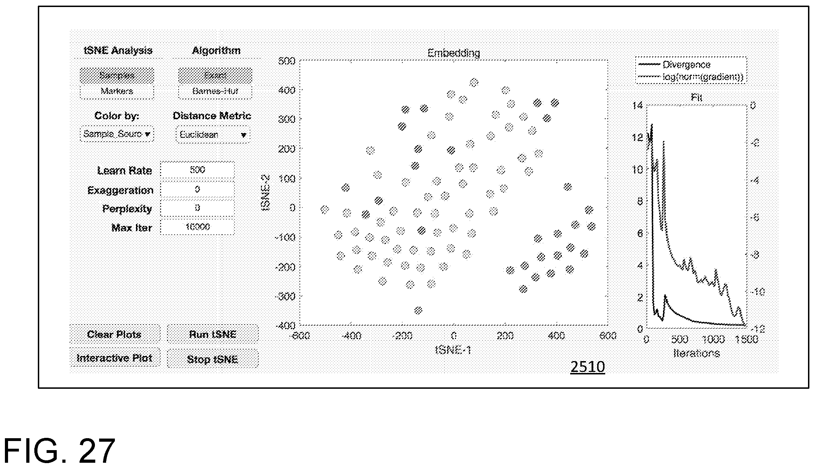

FIG. 25 illustrates a GUI 2500 that can be used for data processing, import, export, and analysis. A series of user activatable regions 2502 (typically responsive to a computer pointing device such as a mouse or track pad) instructs a processor to display heatmaps of various types or a boxplot. A user activatable region 2504 is selectable for normalization based on normalization parameters displayed in a display/entry region 2506. A series 2508 of user activatable regions (conveniently implemented as tabs) permits control of various analysis, acquisition, and display processing. An output or activity region 2510 is shown with display of a histogram and associated control parameters. An additional display region 2512 can show a list of executed commands or other information about prior processing and data import or export procedures. The activity region 2510 can list data files that have been processed, imported, or exported, and a particular display is generally defined in relation to a selection among the series 2508 of tabs. For example, FIG. 26 shows the activity region 2510 with selection of a tab associated with PCA processing. A PCA plot, a scree plot, and PCA display color controls are illustrated. For each tab, the activity region 2510 is generally arranged to display associated data, results of data processing, and provide input for and display of any parameters used in the analysis. In another example, FIG. 27 shows the activity region 2510 with selection of a tab associated with tSNE processing.

### Channel Selection

In the examples described above, sample groups and subgroups are identified based on analyses of channel counts. Such group identifications permit selection of preferred sets of channels for detection of samples in a particular subgroup. For example, the presence of samples associated with particular groups can be identified using channels associated with these groupings, and channel data for other channels need not be acquired. In addition, the identification of useful channels can be used to guide the selection of additional channels.

In view of the many possible embodiments to which the principles of the disclosure may be applied, it should be recognized that illustrated embodiments are only examples and should not be considered a limitation on the scope of the disclosure. We therefore claim all that comes within the scope of the appended claims.

## Claims

1. A method, comprising:
identifying at least two extracellular vesicle (EV) sample groups based on differing responses in multichannel flow cytometry channel counts for a plurality of samples for each of a plurality of channels, each channel defined by a capture agent and a detection agent, and including at least 10 counts;
selecting a set of channels associated with a selected one of the sample groups based on the identified at least two EV sample groups; and
obtaining multichannel flow cytometry channel counts for a test EV sample for each channel of the set of channels to assess whether the test sample is associated with the selected sample group,
wherein the plurality of channels, each channel defined by a capture agent and a detection agent, includes channels identifiable with selected scattered light wavelengths or bands of wavelengths in addition to selected fluorescence wavelengths as well as channels identifiable with scattered light spectra in addition to selected fluorescence spectra,
wherein one or more deconvolution algorithms are used to separate optical signals corresponding to different channels.

2. The method of claim 1, wherein the set of channels is obtained from the multichannel flow cytometry channel counts based on a labeled representation of a t-distributed stochastic neighbor embedding associated with at least some of the plurality of channels.

3. The method of claim 1 or 2, wherein the set of channels is obtained from the multichannel flow cytometry channel counts based on an agglomerative hierarchical clustering or a principal components analysis.

4. The method of any of the preceding claims, wherein the different sets of capture agents range from 40 to 100, and the more than two different sets of detection agents range from 4 to 10.

5. The method of claim 4, wherein the number of channels ranges from 160 to 500.

## Patentansprüche

1. Verfahren, umfassend:
Identifizierung von mindestens zwei extrazellulären Vesikel (EV)-Probengruppen auf der Grundlage unterschiedlichen Ansprechens in Mehrkanal-Durchflusszytometrie-Kanalzählungen für eine Vielzahl von Proben für jeden einer Vielzahl von Kanälen, wobei jeder Kanal durch ein Einfangmittel und ein Nachweismittel definiert ist und mindestens 10 Zählungen enthält;
Auswählen eines Satzes von Kanälen, die mit einer ausgewählten Probengruppe assoziiert sind, auf der Grundlage von den identifizierten mindestens zwei EV-Probengruppen; und
Erhalten von Mehrkanal-Durchflusszytometrie-Kanalzählungen für eine Test-EV-Probe für jeden Kanal des Satzes von Kanälen, um zu beurteilen, ob die Testprobe mit der ausgewählten Probengruppe assoziiert ist,
wobei die Vielzahl von Kanälen, wobei jeder Kanal durch ein Einfangmittel und ein Nachweismittel definiert ist, Kanäle, die mit ausgewählten Streulichtwellenlängen oder Wellenlängenbändern zusätzlich zu ausgewählten Fluoreszenzwellenlängen identifizierbar sind, sowie Kanäle, die mit Streulichtspektren zusätzlich zu ausgewählten Fluoreszenzspektren identifizierbar sind, enthält,
wobei ein oder mehrere Dekonvolutionsalgorithmen verwendet werden, um optische Signale zu trennen, die verschiedenen Kanälen entsprechen.

2. Verfahren nach Anspruch 1, wobei der Satz von Kanälen aus den Mehrkanal-Durchflusszytometrie-Kanalzählungen auf der Grundlage einer beschrifteten Darstellung einer t-verteilten stochastischen Nachbarschaftseinbettung erhalten wird, die mit mindestens einigen der mehreren Kanäle assoziiert ist.

3. Verfahren nach Anspruch 1 oder 2, wobei der Satz von Kanälen aus den Mehrkanal-Durchflusszytometrie-Kanalzählungen auf der Grundlage eines agglomerativen hierarchischen Clustering oder einer Hauptkomponentenanalyse erhalten wird.

4. Verfahren nach einem der vorhergehenden Ansprüche, wobei die verschiedenen Sätze von Einfangmitteln zwischen 40 und 100 liegen und die mehr als zwei verschiedenen Sätze von Nachweismitteln zwischen 4 und 10 liegen.

5. Verfahren nach Anspruch 4, wobei die Anzahl der Kanäle im Bereich von 160 bis 500 liegt.

## Revendications

1. Procédé comprenant:
identifier au moins deux groupes d'échantillons de vésicules extracellulaires (EV) sur la base de réponses différentes dans des comptages de canaux de cytométrie en flux multicanaux pour une pluralité d'échantillons pour chacun d'une pluralité de canaux, chaque canal étant défini par un agent de capture et un agent de détection et comprenant au moins 10 comptages;
sélectionner un ensemble de canaux associés à un groupe d'échantillons sélectionné, sur la base desdits au moins deux groupes d'échantillons EV identifiés ; et
obtenir des comptages de canaux de cytométrie en flux multicanaux pour un échantillon test EV pour chaque canal de l'ensemble de canaux afin d'évaluer si l'échantillon test est associé au groupe d'échantillons sélectionné,
dans lequel la pluralité de canaux, chaque canal défini par un agent de capture et un agent de détection, comprend des canaux identifiables avec des longueurs d'onde de lumière diffusée sélectionnées ou des bandes de longueurs d'onde en plus de longueurs d'onde de fluorescence sélectionnées ainsi que des canaux identifiables avec des spectres de lumière diffusée en plus de spectres de fluorescence sélectionnés,
dans lequel un ou plusieurs algorithmes de déconvolution sont utilisés pour séparer des signaux optiques correspondant à différents canaux.

2. Procédé selon la revendication 1, dans lequel l'ensemble de canaux est obtenu à partir des comptages de canaux de cytométrie en flux multicanaux sur la base d'une représentation étiquetée d'une intégration de voisinage stochastique distribuée en t qui est associée à au moins certains de la pluralité de canaux.

3. Procédé selon la revendication 1 ou 2, dans lequel l'ensemble de canaux est obtenu à partir des comptages de canaux de cytométrie en flux multicanaux sur la base d'un clustering hiérarchique agglomératif ou d'une analyse de composants principaux.

4. Procédé selon l'une quelconque des revendications précédentes, dans lequel les différents ensembles d'agents de capture vont de 40 à 100, et les plus de deux ensembles différents d'agents de détection vont de 4 à 10.

5. Procédé selon la revendication 4, dans lequel le nombre de canaux est compris entre 160 et 500.
